# FASCICULE DE BREVET EUROPEEN

(11) **EP 1 317 921 B1**
(45) Date de publication et mention de la délivrance du brevet: **19.08.2009**
(21) Numéro de dépôt: 01403166.0
(22) Date de dépôt: 07.12.2001
(51) Int. Cl.: A61K 9/08, A61K 9/06, A61K 31/568, A61K 47/14, A61K 47/10, A61P 5/24

(54) **Composition pharmaceutique sous forme de gel ou de solution à base de dihydrotestostérone, son procédé de préparation et ses utilisations**
Gel oder Lösung enthaltend Dihydrotestosterone, deren Herstellungsverfahren und Verwendung
Gel or solution containing dihydrotestosterone, its manufacture and use

(43) Date de publication de la demande: 11.06.2003
(73) Titulaire: Besins International Belgique, 1620 Drogenbos (BE)
(72) Inventeur: Masini-Eteve, Valerie, 91370 Verrieres le Buisson (FR); Taravella, Brigitte, 75014 Paris (FR)
(74) Mandataire: Nargolwalla, Cyra

(56) Documents cités:
- WO-A-97/41865
- US-A- 5 152 997
- US-A1- 2001 036 483
- WANG C ET AL: "Comparative pharmacokinetics of three doses of percutaneous dihydrotestosterone gel in healthy elderly men--a clinical research center study." JOURNAL OF CLINICAL ENDOCRINOLOGY AND METABOLISM, (1998 AUG) 83 (8) 2749-57. , XP002197335
- SOLVAY PHARMACEUTICALS: "AndroGel 1%" INTERNET, [en ligne] septembre 2001 (2001-09), pages 1-2, XP002197336 Extrait de l'Internet: <URL:www.androgel.com/pdfs/ANDROGEL.PDF> [extrait le 2002-04-24]
- BIAM: "Andractim 2.5% Gel pour application locale" INTERNET, [en ligne] 30 novembre 2000 (2000-11-30), pages 1-4, XP002197337 Extrait de l'Internet: <URL:http://www.biam2.org/www/Spe1868.html > [extrait le 2002-04-24]
- KIM M K ET AL: "Skin permeation of testosterone and its ester derivatives in rats." JOURNAL OF PHARMACY AND PHARMACOLOGY, (2000 APR) 52 (4) 369-75. , XP001068685
- SANTUS G C ET AL: "TRANSDERMAL ENHANCER PATENT LITERATURE" JOURNAL OF CONTROLLED RELEASE, ELSEVIER SCIENCE PUBLISHERS B.V. AMSTERDAM, NL, vol. 25, no. 1 / 2, 27 mai 1993 (1993-05-27), pages 1-20, XP000361364 ISSN: 0168-3659

## Description

La présente invention concerne une composition pharmaceutique se présentant sous la forme d'un gel ou d'une solution à base de dihydrotestostérone (DHT). L'invention concerne également les procédés de préparation de ces formulations, ainsi que leurs utilisations.

La DHT est un métabolite de la testostérone. Dans les organes sexuels tels que la prostate et les vésicules séminales, la testostérone est réduite en DHT par une enzyme, la 5-alpha réductase.

Au cours de l'andropause (ou « male menopause » ou « partial deficiency of aging male »), la sécrétion des androgènes diminue, et dans certains cas, ceci peut entraîner des troubles pathogènes. En particulier, on observe une modification de la synthèse protéique et des activités enzymatiques des tissus-cibles. Aux anomalies de production puis de transport de la testostérone, s'ajoutent des anomalies de métabolisme par les tissus-cibles, anomalies qui échappent en majeure partie aux dosages plasmatiques habituellement pratiqués et qui font la spécificité de l'hypogonadisme de l'homme âgé.

Ainsi, chez l'homme âgé andropausé, la testostérone est sécrétée en quantité moindre et surtout son métabolisme en DHT s'effectue de manière insuffisante par les tissus-cibles, suite à un déficit en 5-alpha-réductase, ce qui se traduit par une diminution de l'activité androgénique. Par contre, l'activité aromatasique augmente régulièrement avec l'âge chez la majorité des hommes, ce qui tend à maintenir l'estradiolémie malgré la chute de la testostéronémie. L'importance de ces changements d'activité intra-tissulaire est mise en évidence par une élévation du taux de la SHBG (Serum Hormone Binding Globuline).

La DHT figure parmi les hormones nécessaires au développement des organes génitaux masculins (verge, scrotum, prostate, vésicules séminales). Elle joue également un rôle dans le développement des caractères sexuels secondaires masculins, à savoir la pilosité, le développement de la musculature, la mue de la voix, l'apparition de la libido. Elle a également une action anabolisante sur le squelette et une action stimulante sur la moelle hématopolétique à forte dose.

La DHT est prescrite chez l'homme en traitement systémique des déficits androgéniques généraux survenant en raison d'un hypogonadisme permanent d'origine testiculaire ou hypophysaire, ou d'un hypogonadisme fonctionnel, le plus souvent dû à des interventions chirurgicales, des polytraumatismes, des brûlures, ou des contraintes physiques ou psychiques intenses et prolongées.

La DHT est également utilisée en traitement local chez l'homme en cas de gynécomastie et de lichen scléro-atrophique balano-préputial. Elle peut aussi être prescrite chez la femme en cas de lichen scléro-atrophique vulvaire.

La Société Demanderesse a déjà développé et commercialisé un gel à base de DHT. Cette formulation a fait l'objet d'un brevet FR 2 519 252. La concentration en DHT dans cette formulation est de 2,5%.

La Société Demanderesse est également titulaire d'un brevet EP 0 700 293 concernant l'utilisation de la DHT en androgénothérapie, et revendiquant plus particulièrement les effets favorables de la DHT sur l'hyperplasie de la prostate. Dans ce brevet, il est simplement indiqué qu'un gel hydroalcoolique ayant une teneur en DHT de 0,5 à 3,5% peut être utilisé. Ceci étant, ce brevet ne donne aucune information sur les constituants d'un tel gel, ni sur une quelconque méthode de fabrication, ni sur son efficacité, en particulier dans le cas d'une teneur très inférieure à celle habituellement utilisée de 2,5%. Il n'existe donc pas, à la connaissance de la Société Demanderesse, de gels à base de DHT ayant une teneur en DHT inférieure à 2,5%.

Les applications de gel se font en général une fois par jour, indifféremment le matin ou le soir, en étalant largement le gel sur une grande surface de la peau : bras, épaule, thorax, abdomen ou cuisses, puis en laissant sécher environ cinq minutes avant de remettre un vêtement sur la zone d'application.

En ce qui concerne la formulation sous forme de solution, elle peut être conditionnée sous forme de spray qui se vaporiserait facilement sur une grande surface de peau.

Il s'agit d'une quantité relativement importante de gel ou de solution, à savoir 5 à 10 g par jour, selon l'indication thérapeutique. Le réglage posologique individuel doit également tenir compte de l'intensité du déficit androgénique à compenser ainsi que de la tolérance.

Il serait donc intéressant de pouvoir diminuer la quantité de gel ou de solution à appliquer et de réduire alors la surface d'application. Ceci peut également conduire à une amélioration de l'observance du traitement par le patient et à une diminution du risque de contamination croisée entre deux individus.

En effet, les effets secondaires de la DHT sont loin d'être bénins ; irritabilité, excitation psychomotrice, augmentation de poids, séborrhée, acné, chez l'homme; il y a aussi le problème de la virilisation chez la femme.

En outre, comme pour toute hormone, il est dans l'intérêt des patients de pouvoir réduire la posologie au minimum efficace, afin de réduire les effets non désirés. La Société Demanderesse a également constaté que la formulation de gel selon le brevet FR 2 519 252 contenant de la DHT à raison de 2,5 g par 100 g de gel souffrait d'autres inconvénients tels qu'une relative instabilité physico-chimique pouvant se traduire par l'apparition de cristaux dans le gel lors de la conservation à température ambiante.

La publication dé Wang et al. intitulée « Comparative pharmacokinetics of three doses of percutaneous DHT gel in healthy elderly men-A clinical research center study » (Journal of Clinical Endocrinology and Metabolism, 1998 August, 83 (8) 2749-57) décrit également une étude pharmacocinétique comparant 3 doses d'un gel transdermal de DHT comprenant une teneur en DHT de 0,7%, et des excipients incluant un carbomer, une triéthanolamine, du myristate d'isopropyle, de l'éthanol et de l'eau. L'extrait de l'internet de Solvay Pharmaceuticals intitulée « Androgel 1% », mis en ligne en septembre 2001 décrit un gel de testostérone comprenant de l'éthanol, de l'eau, un carbomer et du myristate d'isopropyle.

La Société Demanderesse a donc cherché à mettre au point une nouvelle formulation à base de DHT permettant de réduire la concentration en DHT à appliquer sur la peau de manière significative, tout en conservant un taux d'absorption cutanée et une efficacité du produit identiques.

Ceci apporte un avantage économique, du fait de la réduction de la concentration en DHT utilisée dans les formulations, en plus des avantages susmentionnés sur le plan thérapeutique et la stabilité physico-chimique de la formulation.

Il est du mérite de la Société Demanderesse d'avoir développé une galénique pour application transdermique à base de DHT pour le traitement de l'homme âgé. En effet, l'utilisation de la DHT dans le traitement de l'hypogonadisme chez l'homme âgé présentant un taux de SHBG anormalement élevé (signe d'un dysfonctionnement intra-tissulaire) est préconisée.

Chez ces hommes, l'activité de l'enzyme 5 α réductase est diminuée. De ce fait, la testostérone est faiblement réduite en DHT, le métabolite actif. Il est donc plus intéressant de traiter les hommes âgés en utilisant directement l'androgène naturellement actif : la DHT.

Il est important de remarquer que les effets estrogéniques de la testostérone ne se manifestent pas sur l'os et pour la plupart des autres cibles. De plus, la testostérone est potentiellement néfaste pour la prostate puisque l'élévation de la testostéronémie est soupçonnée d'élever le risque de cancer chez l'homme âgé (Ly L.P et col., J. Clin. Endocrinol Metab., 2001 ; 86 : 4078-4088 ; Wang C. et col., J. Clin. Endocrinol Metab., 1998 ; 83 : 2749-2757 ; Shaneyfelt T. et col, J. Clin. Oncol. 2000 ; 18 : 847-853) .

Ainsi, l'invention concerne une composition pharmaceutique se présentant sous la forme d'un gel ou d'une solution et **caractérisée en ce qu**'elle contient de la dihydrotestostérone ainsi qu'au moins un promoteur d'absorption percutanée.

On entend par « promoteur d'absorption percutanée », toute molécule favorisant la diffusion d'un principe actif à travers la peau de façon réversible, et tout solubilisant favorisant le partage du principe actif depuis le véhicule vers la couche cornée de l'épiderme.

Selon un mode avantageux de réalisation de la composition pharmaceutique selon l'invention, la teneur en DHT est inférieure à 1,5% et de préférence est de 0,7%, ce pourcentages étant exprimés en poids par rapport à 100g de formulation.

Le solvant mis en oeuvre est un solvant non aqueux, capable de dissoudre la DHT et le promoteur d'absorption. On le choisira parmi des composés de faible point d'ébullition, à savoir inférieur à 100°C à pression atmosphérique, de façon qu'il puisse rapidement s'évaporer au contact de la peau. De tels solvants peuvent être sélectionnés seuls ou en association, parmi des composés volatils tels que l'éthanol, l'isopropanol ou l'acétate d'éthyle ; de préférence l'éthanol et/ou l'isopropanol. Toutefois, l'éthanol représente un solvant préféré selon l'invention puisqu'il contribue avec efficacité au passage transcutané du principe actif en s'évaporant rapidement au contact avec la peau.

De manière à atteindre une concentration efficace en principe actif sans pour autant recouvrir une surface de peau trop importante, on associe au principe actif, un promoteur d'absorption percutanée. Ce dernier entre dans la composition de l'invention à raison de 0,1 à 10 %, de préférence entre 0,3 et 5 %, et plus préférentiellement encore de 0,7%, ces pourcentages étant exprimés en poids pour 100g de formulation.

Ce promoteur d'absorption est choisi de façon à améliorer le passage systémique de la DHT et d'obtenir ainsi les effets désirés moyennant un recouvrement cutané acceptable, c'est à dire inférieur à 15 µg de DHT par cm², de préférence 10 µg de DHT par cm² et plus préférentiellement encore 7 µg de DHT par cm².

Ce promoteur d'absorption cutanée sera sélectionné parmi des substances compatibles avec le solvant non aqueux choisi. Préférentiellement, on le choisira parmi les composés cités ci-dessous qui présentent un degré de solubilité nécessaire dans le solvant en question, non acceptable, c'est à dire inférieur à 15 µg de DHT par cm², de préférence 10 µg de DHT par cm² et plus préférentiellement encore 7 µg de DHT par cm².

Ce promoteur d'absorption cutanée sera sélectionné parmi des substances compatibles avec le solvant non aqueux choisi. Préférentiellement, on le choisira parmi les composés cités ci-dessous qui présentent un degré de solubilité nécessaire dans le solvant en question, non irritant, non allergénique, et non toxique. Le promoteur choisi devra également être compatible avec l'ensemble des composants de la formulation retenue et doit être chimiquement et physiquement stable.

A titre d'exemples de promoteurs susceptibles d'être utilisés seul ou en association dans la composition pharmaceutique selon l'invention et ayant montré de bonnes propriétés pour promouvoir l'absorption cutanée de substances actives on peut citer : les esters d'acide gras aliphatiques comme le myristate d'isopropyle; les acides gras comme l'acide oléique ; les alcools ou polyols tels que l'éthanol, le propylèneglycol et les polyéthylèneglycols ; les composants des huiles essentielles et dérivés terpéniques (comme l'eugenol, le géraniol, le nérol, l'eucalyptol, le menthol) ; les tensioactifs; les hydratants comme la glycérine, l'urée ; des kératolytiques comme les alphahydroxyacides.

Le myristate d'isopropyle représente le promoteur d'absorption préféré pour la composition pharmaceutique selon l'invention.

Dans le cas où la formulation est sous forme de gel, la composition pharmaceutique selon l'invention contient également un agent gélifiant. La composition pharmaceutique sous forme de gel selon l'invention présente alors une teneur entre 0,05 et 3,0 % d'un agent gélifiant, de préférence entre 0,2 et 2 % et plus préférentiellement encore de 0,5%, ces pourcentages étant exprimés en poids pour 100g de gel. Les carbomères, les dérivés cellulosiques, les poloxamères, les poloxamines ou d'autres agents gélifiants seul ou en association peuvent être utilisés dans la formulation sous la forme d'un gel selon l'invention.

Les carbomères sont des polymères acryliques. Ils peuvent être utilisés en tant qu'agents de suspension ou pour augmenter la viscosité des formulations gel.

Un carbomère particulièrement préféré dans le cadre de la présente invention est le Carbopol® 980.

Selon un autre mode avantageux de réalisation de la composition pharmaceutique selon l'invention lorsqu'elle est sous forme d'un gel et en présence de certains types de gélifiant et préférentiellement ceux contenant des fonctions carboxyliques (-COOH) tels que les carbomères, elle peut contenir un agent neutralisant. Le rapport neutralisant/gélifiant est compris entre 10/1 et 0.1/1, préférentiellement entre 7/1 et 0.5/1, et plus préférentiellement est de 1/1. Cet agent neutralisant est choisi de telle manière qu'il forme en présence du polymère des sels qui soient solubles dans le solvant. L'agent neutralisant est également choisi de façon à permettre d'atteindre un gonflement optimal des chaînes de polymère lors de la neutralisation des charges et de la formation de sels de polymères. Selon l'invention, la triéthanolamine est utilisée de préférence comme agent neutralisant en présence de Carbopol® 980. Elle permet également d'atteindre une viscosité optimale dans la composition pharmaceutique selon l'invention. D'autres agents neutralisants comme l'hydroxyde de sodium, l'hydroxyde d'ammonium, l'hydroxyde de potassium, l'arginine, l'aminométhyle de propanol, la trométhamine peuvent être utilisés seul ou en association dans la préparation. L'agent neutralisant est choisi en fonction du type de gélifiant utilisé, d'une manière connue par l'homme du métier.

En androgénothérapie générale, la dose quotidienne habituelle de la composition pharmaceutique sous la forme d'un gel ou d'une solution selon l'invention est comprise entre 2,5 et 5 g de la formulation par jour.

L'invention concerne également un procédé de préparation de la composition pharmaceutique sous la forme d'un gel ou d'une solution selon l'invention.

Ce procédé comporte les étapes successives suivantes :
■ On réalise, sous agitation, une dissolution de la DHT dans un mélange de solvant et de promoteur d'absorption ;
■ On ajoute, sous agitation, de l'eau au mélange obtenu ;

Lorsqu'il s'agit d'un gel, on ajoute les étapes suivantes :
■ On incorpore ensuite au mélange et sous agitation un agent gélifiant, tel que le carbopol ;
■ Eventuellement, on ajoute au mélange, sous agitation, un agent neutralisant tel que la triéthanolamine.

L'invention concerne également l'utilisation du gel ou de la solution selon l'invention pour la préparation d'un médicament pour application par voie transdermique pour le traitement d'une condition physiologique liée à un déficit androgénique.

On peut citer comme exemples de telles conditions physiologiques :
■ Chez l'adulte : l'hypogonadisme permanent ou fonctionnel avec ou sans dysfonctionnement sexuel et/ou avec dépression chez l'homme, l'hyperplasie de la prostate, la gynécomastie, le lichen scléro-atrophique balano-préputial chez l'homme et le lichen scléro-atrophique vulvaire et périanal chez la femme.
■ En pédiatrie : le micropénis.

La composition pharmaceutique selon l'invention peut également comprendre un oestrogène, de préférence sélectionné dans le groupe constitué par le 17β-estradiol, l'estrone, le 17α-éthinyl-estradiol et le valérianate d'estradiol, et plus préférentiellement encore le 17β-estradiol à une dose bioéquivalente à 0,5 mg de 17β-estradiol administré par voie orale.

La DHT possède un effet antigonadotrope et donc anti-estrogène. Bien qu'il ne soit pas prouvé que les estrogènes jouent un rôle positif sur l'os, il pourra être préconisé, à titre préventif, d'associer l'administration d'estrogène à celle de la DHT chez les hommes présentant une estradiolémie insuffisante afin de compenser cette perte et de leur permettre de retrouver un taux physiologique acceptable.

L'invention sera mieux comprise à l'aide des exemples non-limitatifs décrits ci-dessous.

### EXEMPLE I : COMPOSITION PHARMACEUTIQUE SOUS LA FORME D'UN GEL SELON L'INVENTION

Un gel selon l'invention ayant la formulation suivante a été préparé par la Société Demanderesse. Les quantités sont données pour 100 g de gel :

| | |
|---|---|
| Dihydrotestostérone | 0,7g |
| Ethanol à 95% | 71,0g |
| Carbopol 980 | 0,5g |
| Myristate d'Isopropyle | 0,7g |
| Triéthanolamine | 0,5g |
| Eau purifiée qsp | 100,0g |

### EXEMPLE II : COMPOSITION PHARMACEUTIQUE SOUS LA FORME D'UNE SOLUTION SELON L'INVENTION

Une solution selon l'invention ayant la formulation suivante a été préparé par la Société Demanderesse. Les quantités sont données pour 100 g de solution :

| | |
|---|---|
| Dihydrotestostérone | 0,7g |
| Ethanol à 95% | 71,0g |
| Myristate d'Isopropyle | 0,7g |
| Eau purifiée qsp | 100,0g |

### EXEMPLE III : PROCEDE DE PREPARATION D'UN GEL SELON L'INVENTION

La fabrication d'un gel à base de DHT selon l'invention se fait comme suit : Pour un lot de 70 Kg à 0,7% de DHT, on procède de la manière suivante :

Dans la cuve d'un mélangeur de type Koruma™, on ajoute sous vide de 800 mbars sans agiter 49700 g d'éthanol à 95%. Puis, par le dessus de la cuve, on ajoute 490 g de myristate d'isopropyle. Enfin, on ajoute, par le dessus de la cuve 490 g de DHT.

On mélange pendant 10 minutes, turbine à 2000 t/min, racleur à 40t/min, jusqu'à dissolution complète de la DHT.

On ajoute 18620 g d'eau purifiée sous vide de 800 mbars et on mélange avec un racleur à 40 t/min.

On ajoute 350 g de carbopol®980 sous vide à 800 mbars. On mélange à 2000 t/min. On arrête le vide. On mélange pendant 10 minutes, turbine à 2000 t/min, racleur à 40 t/min.

On ajoute la triéthanolamine par le dessus de la cuve. On mélange pendant 3 min, turbine à 2000 t/min, racleur à 40 t/min.

On met le mélangeur sous vide à 120 mbars pendant 2 à 3 minutes. Ensuite, on arrête le vide puis on agite pendant 20 minutes avec le racleur à 40 t/min.

### EXEMPLE IV : PROCEDE DE PREPARATION D'UNE SOLUTION SELON L'INVENTION

La fabrication d'une solution à base de DHT selon l'invention se fait comme suit

Pour un lot de 70 Kg à 0,7% de DHT, on procède de la manière suivante :

Dans la cuve d'un mélangeur de type Koruma™, on ajoute sous vide de 800 mbars sans agiter, 49700 g d'éthanol à 95%. Puis, par le dessus de la cuve, on ajoute 490 g de myristate d'isopropyle. Enfin, on ajoute, par le dessus de la cuve 490 g de DHT.

On mélange pendant 10 minutes, turbine à 2000 t/min, racleur à 40t/min, jusqu'à dissolution complète de la DHT.

On ajoute 19320 g d'eau purifiée sous vide de 800 mbars, racleur à 40 t/min.

On met le mélangeur sous vide à 120 mbars pendant 2 à 3 minutes. Ensuite, on arrête le vide puis on agite pendant 20 minutes avec le racleur à 40 t/min.

### EXEMPLE V : ETUDES PHARMACOCINETIQUES IN VITRO ET IN VIVO

### Etudes in vitro :

Des études d'absorption percutanée comparatives sur peau abdominale humaine entre la formulation ANDRACTIM® à 2,5% de DHT et la formulation de gel à 0,7% décrite selon l'invention ont été réalisées sur cellules de Franz.

Les formulations ont été appliquées à une dose d' environ 13 µg de DHT par cm² sur une surface cutanée de 1,77 cm².

Les résultats montrent que les taux de DHT absorbée sont comparables entre les deux formulations : 0,47 µg et 0,32 µg de DHT absorbée, respectivement pour le gel à 0,7% et le gel à 2,5%.

### Etude in vivo :

Une étude pharmacocinétique de phase I a été réalisée afin de comparer les paramètres pharmacocinétiques de la formulation ANDRACTIM® à 2,5% de DHT et la formulation selon l'invention à 0,7% de DHT après administration percutanée répétée. Cette étude était une étude ouverte sur 18 patients sans placebo et réalisée en cross-over. 5 g de gel ANDRACTIM® à 2,5% (soit 125 mg de DHT) ou 5 g de gel selon l'invention à 0,7% (soit 35 mg de DHT) ont été administrés une fois par jour pendant 7 jours.

Les paramètres pharmacocinétiques de chacune des formulations administrées ont été évalués et sont les suivants.
■ Les concentrations plasmatiques moyennes en DHT observées en fonction de la formulation mise en oeuvre sont résumées dans le tableau ci-dessous aux jours 1, 5 et 8 suivant le début de l'étude (jour 0)..

**TABLEAU I**

| | **Moyenne ± écart type (ng/mL)** | | |
|---|---|---|---|
| | **Jour 1** | **Jour 5** | **Jour 8** |
| **ANDRACTIM 2,5%** | 0.597±0.165 | 4.31±2.06 | 3.62±1.83 |
| **DHT gel 0,7%** | 0.621±0.217 | 3.83±2.02 | 3.38±1.30 |

■ Les AUC (Area Under Curve) calculées entre 0 et 24 heures au jour 7 sont égales à 85,4 ng.h/mL (CV de 35%) après traitement avec ANDRACTIM® 2,5% et de 102 ng.h/mL (CV de 33%) après traitement avec la formulation de gel DHT selon l'invention à 0,7%.
■ Les concentrations plasmatiques moyennes de DHT au jour 7 (de 0 à 24 heures) sont égales à 3,98 ± 1,32 ng/mL après traitement avec ANDRACTIM® 2,5% et à 4,60 ± 1,51 ng/mL après traitement avec la formulation de gel DHT selon l'invention à 0,7%.

L'ensemble des résultats obtenus *in vivo* montre que les deux traitements (ANDRACTIM® 2,5% et la formulation de gel DHT selon l'invention à 0,7%) présentent des pharmaco-cinétiques relativement similaires.

Les tests statistiques réalisés en accord avec la réglementation internationale en vigueur sur les médicaments destinés à être administrés chez l'homme démontrent que la bioéquivalence n'est pas significative entre les deux traitements et que la formulation DHT 0.7% selon l'invention est suprabiodisponible par rapport à ANDRACTIM® 2,5%.

## Revendications

1. Composition pharmaceutique se présentant sous la forme d'un gel ou d'une solution présentant une teneur en dihydrotestostérone inférieure à 1,5%, présentant une teneur entre 0,1 et 10 %, d'un promoteur d'absorption percutanée, et présentant une teneur en solvant comprise entre 30 et 85 %, ces pourcentages étant exprimés en poids par rapport à 100g de formulation.

2. Composition pharmaceutique selon la revendication 1, présentant une teneur en dihydrotestostérone inférieure à 0,7%, ce pourcentage étant exprimé en poids par rapport à 100g de formulation.

3. Composition pharmaceutique selon l'une ou l'autre des revendications 1 et 2, présentant une teneur en solvant comprise entre 40 et 75 %, et plus préférentiellement encore de 71 %, ces pourcentages étant exprimés en poids par rapport à 100g de formulation.

4. Composition pharmaceutique selon l'une quelconque des revendications 1 à 3, dans laquelle le solvant est sélectionné dans le groupe constitué par l'éthanol, l'isopropanol et l'acétate d'éthyle, ainsi que leurs mélanges, de préférence étant l'éthanol et/ou l'isopropanol.

5. Composition pharmaceutique selon l'une quelconque des revendications 1 à 4, présentant une teneur entre 0,3 et 5 %, et plus préférentiellement encore de 0,7% d'un promoteur d'absorption percutanée, ces pourcentages étant exprimés en poids par rapport à 100g de formulation.

6. Composition pharmaceutique selon l'une quelconque des revendications 1 à 5, dans laquelle le promoteur d'absorption percutanée est sélectionné dans le groupe constitué par les esters d'acide gras aliphatiques comme le myristate d'isopropyle; les acides gras comme l'acide oléique ; les alcools ou polyols tels que l'éthanol, le propylèneglycol et les polyéthylèneglycols ; les composants des huiles essentielles et dérivés terpéniques (comme l'eugenol, le géraniol, le nérol, l'eucalyptol, le menthol) ; les tensiocatifs; les hydratants comme la glycérine, l'urée ; des kératolytiques comme les alphahydroxyacides, ainsi que leurs mélanges, et de préférence est le myristate d'isopropyle.

7. Composition pharmaceutique selon l'une quelconque des revendications 1 à 6, présentant une teneur entre 0,05 et 3 % d'un agent gélifiant, de préférence entre 0,2 et 2 % et plus préférentiellement encore de 0,5%, ces pourcentages étant exprimés en poids par rapport à 100g de formulation.

8. Composition pharmaceutique selon la revendication 7, dans laquelle l'agent gélifiant est sélectionné dans le groupe constitué par les carbomères, les dérivés cellulosiques, les poloxamères, les poloxamines, ainsi que leurs mélanges, de préférence, les carbomères.

9. Composition pharmaceutique selon l'une ou l'autre des revendications 7 et 8, comprenant en outre un agent neutralisant.

10. Composition pharmaceutique selon la revendication 9, dans laquelle l'agent neutralisant est sélectionné dans le groupe constitué par la triéthanolamine, l'hydroxyde de sodium, l'hydroxide d'ammonium, l'hydroxide de potassium, l'arginine, l'aminométhyle de propanol, la trométhamine, ainsi que leurs mélanges, étant de préférence la triéthanolamine.

11. Composition pharmaceutique selon l'une ou l'autre des revendications 9 et 10, dans laquelle le rapport d'agent neutralisant/agent gélifiant est compris entre 10/1 et 0.1/1, de préférence entre 7/1 et 0.5/1, et plus préférentiellement encore est de 1/1.

12. Procédé de préparation d'une composition pharmaceutique selon l'une quelconque des revendications 1 à 11 , **caractérisé en ce que** :
- on prépare un mélange composé d'un solvant, d'un promoteur de promotion et de la DHT;
- on ajoute éventuellement un agent gélifiant à ce mélange, puis on mélange à nouveau ;
- on ajoute éventuellement un agent neutralisant, puis on mélange à nouveau ;
- on récupère la composition pharmaceutique contenant de la DHT.

13. Utilisation d'une composition pharmaceutique selon l'une quelconque des revendications 1 à 11, ou préparée selon la revendication 12, pour la préparation d'un médicament pour application par voie transdermique pour le traitement des conditions physiologiques liées à l'insuffisance de la dihydrotestostérone.

14. Utilisation selon la revendication 13 dans laquelle les conditions physiologiques sont sélectionnées dans le groupe constitué par l'hypogonadisme permanent, l'hypogonadisme fonctionnel, l'hyperplasie de la prostate, la gynécomastie, le lichen scléro-atrophique balano-préputial chez l'homme et le lichen scléro-atrophique vulvaire chez la femme ou le micropenis chez l'enfant.

## Claims

1. Pharmaceutical composition in the form of a gel or a solution, **characterized in that** it contains a dihydrotestosterone content of less than 1.5%, a content percutaneous absorption promoter of between 0.1% and 10% and a solvent content of between 30% and 85%, these percentages being expressed by weight relative to 100 g of formulation.

2. Pharmaceutical composition according to claim 1, wherein the dihydrotestosterone content is of 0.7%, this percentage being expressed by weight relative to 100 g of formulation.

3. Pharmaceutical composition according to any of claim 1 or 2, with a solvent content of between 40% and 75%, and even more preferably of 71%, these percentages being expressed by weight relative to 100 g of formulation.

4. Pharmaceutical composition according to any of claims 1 to 3, in which the solvent is selected from the group consisting of ethanol, isopropanol and ethyl acetate, as well as mixtures thereof, and is preferably ethanol and/or isopropanol.

5. Pharmaceutical composition according to any of claims 1 to 4, with a content of between 0.3% and 5%, and even more preferably of 0.7% of a percutaneous absorption promoter, these percentages being expressed by weight relative to 100 g of formulation.

6. Pharmaceutical composition according to any of claims 1 to 5, in which the percutaneous absorption promoter is selected from the group consisting of aliphatic fatty acid esters such as isopropyl myristate; fatty acids such as oleic acid; alcohols or polyols such as ethanol, propylene glycol and polyethylene glycols; components of essential oils and terpine derivatives (such as eugenol, geraniol, nerol, eucalyptol or menthol); surfactants; moisturizers such as glycerol, urea; keratolytic agents such as α-hydroxy acids, and also mixtures thereof, and is preferably isopropyl myristate.

7. Pharmaceutical composition according to claim 1, with a content of between 0.05% and 3% of a gelling agent, preferably between 0.2% and 2% and even more preferably of 0.5%, these percentages being expressed by weight relative to 100 g of formulation.

8. Pharmaceutical composition according to claim 7, in which the gelling agent is selected from the group consisting of carbomers, cellulose derivatives, poloxamers, poloxamines and mixtures thereof, preferably carbomers.

9. Pharmaceutical composition according to claim 7, further comprising a neutralizer.

10. Pharmaceutical composition according to claim 9, in which the neutralizer is selected from the group consisting of triethanolamine, sodium hydroxide, ammonium hydroxide, potassium hydroxide, arginine, aminomethylpropanol and tromethamine, and also mixtures thereof, and is preferably triethanolamine.

11. Pharmaceutical composition according to claim 9, in which the neutralizer-gelling agent ratio is between 10/1 and 0.1/1, preferably between 7/1 and 0.5/1, and even more preferably is 1/1.

12. Process for preparing a pharmaceutical composition according to claim 1, **characterized in that:**
- a mixture composed of a solvent, an absorption promoter and DHT is prepared;
- a gelling agent is optionally added to this mixture, and mixing is again carried out;
- a neutralizer is optionally added, and mixing is then again carried out;
- the pharmaceutical composition containing DHT is recovered.

13. Method for the treatment of physiological conditions associated with insufficiency of dihydrotestosterone, comprising the step of administering a pharmaceutical composition according to claim 1 to a subject.

14. Use according to claim 13, in which the physiological conditions are selected from the group consisting of permanent hypogonadism, functional hypogonadism, hyperplasia of the prostate, gynaecomasty, balano-preputial sclero-atrophic lichen in men and vulval sclero-atrophic lichen in women, or micropenis in children.

## Patentansprüche

1. Pharmazeutische Zusammensetzung in Form eines Gels oder einer Lösung, die Folgendes aufweist: einen Gehalt an Dihydrotestosteron unter 1,5%, einen Gehalt zwischen 0,1 und 10% eines Förderers der perkutanen Absorption und einen Gehalt an Lösungsmittel zwischen 30 und 85%, wobei diese Prozentangaben als Gewichtsprozent, bezogen auf 100 g der Formulierung, ausgedrückt sind.

2. Pharmazeutische Zusammensetzung nach Anspruch 1, die einen Gehalt an Dihydrotestosteron von 0,75% auf-weist, wobei diese Prozentangabe als Gewichtsprozent, bezogen auf 100 g der Formulierung, ausgedrückt ist.

3. Pharmazeutische Zusammensetzung nach einem der Ansprüche 1 und 2, die einen Gehalt an Lösungsmittel zwischen 40 und 75% und vorzugsweise von 71% aufweist, wobei diese Prozentangaben als Gewichtsprozent, bezogen auf 100 g der Formulierung, ausgedrückt sind.

4. Pharmazeutische Zusammensetzung nach einem der Ansprüche 1 bis 3, wobei das Lösungsmittel aus der Gruppe ausgewählt ist, bestehend aus Ethanol, Isopropanol und Ethylacetat sowie ihren Gemischen und vorzugsweise Ethanol und/oder Isopropanol ist.

5. Pharmazeutische Zusammensetzung nach einem der Ansprüche 1 bis 4, die einen Gehalt zwischen 0,3 und 5% und vorzugsweise von 0,7% eines Förderers der perkuta-nen Absorption aufweist, wobei diese Prozentangaben als Gewichtsprozent, bezogen auf 100 g der Formulierung, ausgedrückt sind.

6. Pharmazeutische Zusammensetzung nach einem der Ansprüche 1 bis 5, wobei der Förderer der perkutanen Absorption aus der Gruppe ausgewählt ist, bestehend aus aliphatischen Fettsäureestern, wie Isopropylmyristat; Fettsäuren, wie Oleinsäure; Alkoholen oder Polyolen, wie Ethanol, Propylenglykol und Polyethylenglykolen; Bestandteilen essenzieller Öle und Terpenderivaten (wie Eugenol, Geraniol, Nerol, Eukalyptol, Menthol); oberflächen-aktiven Mitteln; Hydratationsmittel, wie Glycerin, Harnstoff; Keratolytika, wie alpha-Hydroxysäuren, sowie deren Gemischen, und vorzugsweise Isopropylmyristat ist.

7. Pharmazeutische Zusammensetzung nach einem der Ansprüche 1 bis 6, die einen Gehalt zwischen 0,05 und 3% eines gelbildenden Mittels, vorzugsweise zwischen 0,2 und 2% und noch stärker bevorzugt von 0,5% aufweist, wobei diese Prozentangaben als Gewichtspro-zent, bezogen auf 100 g der Formulierung, ausgedrückt sind.

8. Pharmazeutische Zusammensetzung nach Anspruch 7, wobei das gelbildende Mittel aus der Gruppe, bestehend aus Carbomeren, Cellulosederivaten, Poloxameren, Polox-aminen sowie ihren Gemischen, vorzugsweise aus Carbo-meren ausgewählt ist.

9. Pharmazeutische Zusammensetzung nach einem der Ansprüche 7 und 8, die außerdem ein Neutralisations-mittel umfasst.

10. Pharmazeutische Zusammensetzung nach Anspruch 9, wobei das Neutralisationsmittel aus der Gruppe ausge-wählt ist, bestehend aus Triethanolamin, Natrium-hydroxid, Ammoniumhydroxid, Kaliumhydroxid, Arginin, Aminomethylpropanol, Tromethamin sowie ihren Gemischen, und vorzugsweise Tromethamin ist.

11. Pharmazeutische Zusammensetzung nach einem der Ansprüche 9 und 10, wobei das Verhältnis Neutralisa-tionsmittel/gelbildendes Mittel zwischen 10/1 und 0,1/1, vorzugsweise zwischen 7/1 und 0,5/1 beträgt und noch stärker bevorzugt 1/1 ist.

12. Verfahren zur Herstellung einer pharmazeutischen Zusammensetzung nach einem der Ansprüche 1 bis 11, **dadurch gekennzeichnet, dass** man:
a) ein Gemisch herstellt, das aus einem Lösungs-mittel, einem Promotionsförderer und DHT besteht;
b) gegebenenfalls ein gelbildendes Mittel zu diesem Gemisch hinzu gibt und dann erneut mischt;
c) gegebenenfalls ein Neutralisationsmittel hinzu gibt und dann erneut mischt;
d) die pharmazeutische Zusammensetzung gewinnt, die DHT enthält.

13. Verwendung einer pharmazeutischen Zusammensetzung nach einem der Ansprüche 1 bis 11 oder hergestellt nach Anspruch 12 zur Herstellung eines Arzneimittels für die transdermale Anwendung zur Behandlung physiologischer Zustände, die mit einem Dihydrotestosteron-Mangel einhergehen.

14. Verwendung nach Anspruch 13, wobei die physio-logischen Zustände aus der Gruppe ausgewählt sind, bestehend aus permanentem Hypogonadismus, funktionellem Hypogonadismus, Prostatahyperplasie, Gynäkomastie, balanopräputialem Lichen sclerosus et atrophicus beim Mann und vulvärem Lichen sclerosus et atrophicus bei der Frau oder Mikropenis beim Kind.
